# EUROPÄISCHE PATENTSCHRIFT

(11) **EP 1 130 013 B1**
(45) Veröffentlichungstag und Bekanntmachung des Hinweises auf die Patenterteilung: **29.10.2003**
(21) Anmeldenummer: 01810209.5
(22) Anmeldetag: 01.03.2001
(51) Int. Cl.: C07C 255/24, C07C 253/16, C07C 253/32, C07C 253/00

(54) **Farbstabile Lösung von Dimethylaminoacetonitril in Wasser und Verfahren zu ihrer Herstellung**
Colour stabile solution of dimethylaminoacetonitrile in water and method for the preparation thereof
Solution de couleur stable de diméthylaminoacétonitrile dans l'eau et procédé pour sa préparation

(30) Priorität: 03.03.2000 DE 10010593
(43) Veröffentlichungstag der Anmeldung: 05.09.2001
(73) Patentinhaber: Clariant GmbH, 65929 Frankfurt am Main (DE)
(72) Erfinder: Fell, Rainer, 86462 Langweid (DE); Wilbert, Götz, 86368 Gersthofen (DE); Stährfeldt, Thomas, 79576 Weil am Rhein (DE)
(74) Vertreter: D'haemer, Jan Constant

(56) Entgegenhaltungen:
- DE-A- 2 503 582
- DE-A- 2 555 769
- DE-A- 2 742 911
- DE-A- 2 748 964
- FR-E- 95 268
- PATENT ABSTRACTS OF JAPAN vol. 003, no. 072 (C-049), 21. Juni 1979 (1979-06-21) & JP 54 046720 A (SHOWA DENKO KK), 12. April 1979 (1979-04-12)

## Beschreibung

Die vorliegende Erfindung bezieht sich auf eine farbstabile Lösung von Dimethylaminoacetonitril in Wasser sowie auf ein Verfahren zu ihrer Herstellung.

Die Verbindungsklasse der Aminoacetonitrile ist bereits seit vielen Jahrzehnten bekannt (W. Eschweiler, Annalen 1894, 279, 34-44). Die technische Produktion von Vertretern dieser Substanzklasse wurde z. B. in DE-A-2503582 beschrieben. Diese Verbindungen stellen wertvolle Ausgangsmaterialien für diverse Feinchemikalien dar. Beispielsweise können ausgehend von diesen Produkten verschiedene Derivate von Glycin aufgebaut werden. So kann aus N,N-Dimethylaminoacetonitril durch Verseifen mit KOH das N,N-Dimethylglycin-Kaliumsalz gewonnen werden (DE-A-2503582). Durch Reaktion von N,N-Dimethylaminoacetonitril mit Chlor entsteht das Tetrachlorethylen-bis-isocyaniddichlorid, eine Ausgangsverbindung für das Fungizid 2-Methylimino-3-(4'-chlorphenyl)-4,5-bis-(trifluormethylimino)-thiazolidin (DE-A-2748964). N,N-Dimethylaminoacetonitril ist auch ein Synthesebaustein von α-Sinensal, ein Sesquiterpenaldehyd, das als Aromastoff eingesetzt wird (GB-A-1467751).

Neben dem Amin und flüssiger Blausäure wird bei der Herstellung von Dimethylaminoacetonitril (nachfolgend DMAA genannt) eine Formaldehyd-Quelle (Formalin, Paraformaldehyd) eingesetzt. Gewöhnlich wird eine wässrige Formaldehyd-Lösung langsam mit flüssiger Blausäure und einer wässrigen Dimethylaminlösung zusammengeführt [1. Schritt der Aminosäuresynthese nach Strecker; (H. Beyer, Lehrbuch der Organischen Chermie, 23. Auflage, Hirzel Verlag, Stuttgart, 1998, S. 302)]. Die Reaktion kann in einem Rührkessel oder alternativ in einer kontinuierlich betriebenen Mischapparatur durchgeführt werden.

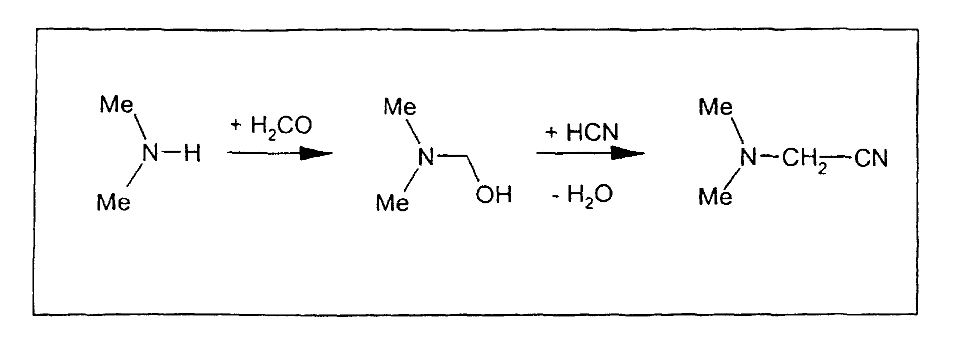

Weiterhin führt auch die Umsetzung von Paraformaldehyd mit flüssiger Blausäure und einer wässrigen Dimethylaminlösung zum gewünschten Endprodukt. Hier ist zu beachten, dass der Einsatz eines Feststoffes (Paraformaldehyd) in der Technik gewöhnlich zu einem unerwünschten und kostentreibenden Mehraufwand führt, welcher bei der Synthese von Glycin in Kauf genommen wird (Ullmanns Encyclopädie der technischen Chemie, Band 7, 4. Auflage, 1974, VCH, Weinheim, S. 432).

Nach der Umsetzung fällt eine wässrige Lösung von DMAA an. Als weitere Chemikalien können sich in meist geringer Konzentration das eingesetzte Amin, Formalinreste, HCN, das Zwischenprodukt Dimethylaminomethanol, Bis-(dimethylamino)methan und diverse Stabilisatoren im Endprodukt befinden. Bei den angesprochenen Stabilisatoren handelt es sich beispielsweise um Stoffe, mit denen die wässrige Formaldehyd-Lösung stabilisiert ist. In vielen Fällen kommt der Reinheit der DMAA-Lösung eine entscheidende Bedeutung zu. So sollten die Konzentrationen insbesondere der gesundheitsschädlichen Edukte und Nebenprodukte so gering wie möglich sein.

Weiterhin kann festgestellt werden, dass die Qualität der DMAA-Lösung beim Lagern abnimmt. Hier ist besonders die Farbe der Lösung ein Merkmal, das sich bei der Lagerung verschlechtert. In der Technik wird daher gewöhnlich die DMAA-Lösung direkt weiterverarbeitet oder vor einer Lagerung destilliert. Ersteres ist aus logistischen Gründen oft nicht möglich und letzteres ist umständlich und verteuert den Preis der DMAA-Lösung deutlich.

Es besteht daher ein Bedarf an einem kostengünstigen Verfahren, bei welchem eine sehr reine DMAA-Lösung direkt in einer lagerstabilen Form anfällt.

Überraschenderweise wurde gefunden, dass man eine farbstabile wässrige Lösung von DMAA erhält, wenn man die wässrige Formaldehyd-Lösung mit 2,4-Diamino-6-phenyl-1,3,5-triazin (Benzoguanamin) stabilisiert.

Gewöhnlich wird eine wässrige Formaldehyd-Lösung mit einem Alkohol - meist mit Methanol - stabilisiert. Wird die Formaldehyd-Lösung jedoch mit Benzoguanamin stabilisiert, so bildet sich bei schonender Umsetzung dieser Lösung mit Dimethylamin (vorzugsweise in wässriger Lösung) und flüssiger Blausäure eine DMAA-Lösung, die ohne weitere Aufarbeitung sehr rein und lagerstabil ist. Eine erfindungsgemäss hergestellte DMAA-Lösung ist in ihrer Lagerstabilität einer destillierten DMAA-Lösung ebenbürtig und einer konventionell hergestellten Lösung insbesondere betreffs der Farbstabilität deutlich überlegen.

Der Stabilisator Benzoguanamin wird besonders vorteilhafterweise in einer Konzentration von 0.05 bis 0.7 Gew.-%, bezogen auf Formaldehyd, eingesetzt.

Eine geeignete DMAA-Lösung wird dann erhalten, wenn das Dimethylamin mit flüssiger Blausäure und einer Formaldehyd-Quelle (Formalin oder Paraformaldehyd) in Anwesenheit von 2,4-Diamino-6-phenyl-1,3,5-triazin in Gegenwart von Wasser während 5 min bis 5 Stunden bei einer Temperatur von 0 bis 50 °C umgesetzt wird. Wenn die Formaldehyd-Quelle vorgelegt wird und nach portionsweiser Zugabe der wässrigen Aminlösung als Zwischenverbindung das Dimethylaminomethanol hergestellt wird, kann anschließend die flüssige Blausäure recht zügig zugegeben werden. In beiden Schritten sollte allerdings die Temperatur 50 °C nicht überschreiten, besonders vorteilhaft wird zwischen 25 und 35 °C gearbeitet. Die Umsetzung eignet sich auch für eine kontinuierlich betriebene Reaktion. Auch hier sollte die Reaktionstemperatur unter 50 °C bleiben.

Die Ausgangssubstanzen können in stöchiometrischer Menge eingesetzt werden oder auch in leichtem Überschuss, vorzugsweise werden 0.98 - 1,02 Mol Formaldehyd und 0.95 - 1.10 Mol Dimethylamin je Mol Blausäure eingesetzt.

Gegenstand der Erfindung ist somit ein Verfahren zur Herstellung einer wässrigen DMAA-Lösung ausgehend von einer Formaldehyd-Quelle, insbesonders einer wässrigen Formaldehydlösung, Dimethylamin und Blausäure, wobei das Reaktionsgemisch bereits vor der Umsetzung 2,4-Diamino-6-phenyl-1,3,5-triazin (Benzoguanamin) in einer Konzentration von 0.001 bis 5 Gew.-%, bezogen auf Formaldehyd, enthält und die Ausgangssubstanzen stöchiometrisch oder in einem Verhältnis von 0.98 - 1.02 Mol Formaldehyd und 0.95 - 1.10 Mol Dimethylamin je Mol Blausäure eingesetzt werden.

Als Formaldehyd-Quelle kann Paraformaldehyd oder Formaldehyd in gasförmiger oder flüssiger Form oder Formaldehyd in seiner wässrigen Lösung verwendet werden. In letzterem Fall ist eine Formaldehyd-Konzentration von 20 bis 50 Gew.-% im Wasser vorteilhaft, insbesondere eignet sich eine wässrige Lösung mit einem Gehalt an Formaldehyd von 30 bis 40 Gew.-%. Das Dimethylamin kann in flüssiger oder gasförmiger Form oder zweckmässigerweise in wässriger Lösung eingesetzt werden, wobei das Dimethylamin in der wässrigen Lösung in einer Konzentration zwischen 20 und 80 Gew.-% vorliegt und besonders vorteilhafterweise eine Konzentration von 35 bis 65 Gew.-% aufweist.

Die überraschende Wirkung des erfindungsgemäss eingesetzten Stabilisators ist auf eine wässrige DMAA-Lösung beschränkt, da andere Amine nur wenig wasserlösliche Aminoacetonitrile bilden.

Es könnte vermutet werden, dass die ungenügende Lagerstabilität der konventionellen DMAA-Lösung auf die Anwesenheit von Methanol als Formalin-Stabilisator zurückgeführt werden kann. Die Anwesenheit von Methanol kann auch alternativ durch den Einsatz von Paraformaldehyd vermieden werden. Diese Alternative ist durch die Problematik der Feststoffverarbeitung mit einem höheren Aufwand verbunden, der nicht erwünscht ist. Eine mit Paraformaldehyd hergestellte recht saubere DMAA-Lösung weist zwar direkt nach der Synthese eine sehr gute Farbe auf (die durchaus mit einer destillierten Lösung vergleichbar ist), neigt aber ebenfalls zu einem deutlichen Nachdunkeln bei der Lagerung.

Es kann somit festgestellt werden, dass eine 2,4-Diamino-6-phenyl-1,3,5-triazinhaltige DMAA-Lösung deutlich farbstabiler ist als eine mit Paraformaldehyd hergestellte 2,4-Diamino-6-phenyl-1,3,5-triazin-freie Lösung. Nur dann, wenn die DMAA-Lösung aufwendig destilliert wurde (wobei 47% DMAA mit 53% Wasser als Azeotrop übergeht; R. A. Turner, J. Am. Chem. Soc. 1946, 68, 1607-1608), kann auf die Anwesenheit von 2,4-Diamino-6-phenyl-1,3,5-triazin verzichtet werden. Die Synthese in Anwesenheit von 2,4-Diamino-6-phenyl-1,3,5-triazin bewirkt also eine ähnlich gute Farbstabilität, wie sie durch eine Destillation erreicht werden kann, erspart aber diesen zusätzlichen und aufwendigen Verfahrensschritt.

Die Erfindung betrifft daher auch eine DMAA-Lösung, die 2,4-Diamino-6-phenyl-1,3,5-triazin enthält. Die Konzentration an 2,4-Diamino-6-phenyl-1,3,5-triazin in der DMAA-Lösung liegt zwischen 0.001 und 2.0 Gew.-%, bezogen auf das DMAA, vorzugsweise zwischen 0.01 und 0.25 Gew.-%.

Die folgenden Beispiele sollen die Erfindung näher erläutern. Die Angaben in % sind dabei als Gew.-% zu verstehen.

### Beispiele:

### Vergleichsbeispiel 1:

### Herstellung einer wässrigen DMAA-Lösung aus Methanol-stabilisiertem Formaldehyd:

83 g Formalin (36%ige Lösung in Wasser mit Methanol stabilisiert; 1.00 mol Formaldehyd) werden vorgelegt und 77 g Dimethylaminlösung (60%ige Lösung in Wasser; 1.02 mol Dimethylamin) innerhalb von 20 min zugetropft (T < 35 °C). Die Lösung wird auf ca. 20°C gekühlt, 27 g (1.00 mol) Blausäure portionsweise zugegeben (T < 35 °C) und 1 h bei ca. 20°C nachgerührt. Die Produktlösung enthält 45% DMAA in Wasser, die ohne zusätzliche Aufarbeitung als Rohstoff für aufbauende Synthesen weiter verwendet werden kann.
Im folgenden bedeuten: JFZ = Jodfarbzahl nach DIN 6162; L*ab = Farbmesszahlen nach DIN 6174

Die Werte wurden direkt nach der Synthese und nach Dunkellagerung im verschlossenen Glasgefäss nach unterschiedlichen Lagerzeiten gemessen.

| **Messzeitpunkt** | **JFZ** | **L**^{**∗**} | **a** | **b** |
|---|---|---|---|---|
| direkt nach Synthese | 0,2 | 100,1 | -0,7 | 1,7 |
| Dunkellagerung 4 Tage | 0,3 | 100,3 | -1,1 | 2,6 |
| Dunkellagerung 10 Tage | 0,5 | 100,0 | -1,7 | 4,4 |
| Dunkellagerung 30 Tage | 1,4 | 98,7 | -3,2 | 8,7 |

### Vergleichsbeispiel 2:

### Herstellunq einer wässrigen DMAA-Lösung aus Paraformaldehyd:

77 g Dimethylaminlösung (60%ige Lösung in Wasser; 1.02 mol Dimethylamin) werden vorgelegt und 31 g Paraformaldehyd werden portionsweise zugegeben (T < 35 °C). Die Lösung wird auf ca. 20 °C gekühlt und 27 g (1.00 mol) Blausäure portionsweise zugegeben (T < 35 °C). Die Lösung wird für 5 min auf ca. 40 °C erwärmt, um das Paraformaldehyd vollständig aufzulösen und 1 h bei ca. 20 C nachgerührt.
Die Produktlösung enthält 63% DMAA in Wasser, die ohne zusätzliche Aufarbeitung als Rohstoff für aufbauende Synthesen weiter verwendet werden kann.

| **Messzeitpunkt** | **JFZ** | **L∗** | **a** | **b** |
|---|---|---|---|---|
| direkt nach Synthese | 0,1 | 100,1 | -0,3 | 0,9 |
| Dunkellagerung 4 Tage | 0,1 | 100,2 | -0,5 | 1,4 |
| Dunkellagerung 10 Tage | 0,4 | 99,9 | -1,1 | 3,1 |
| Dunkellagerung 30 Tage | 1,1 | 99,7 | -3,2 | 8,1 |

### Vergleichsbeispiel 3:

### Herstellung einer destillativ gereinigten wässrigen DMAA-Lösung:

Produktlösung aus Vergleichsbeispiel 1 wird bei Normaldruck fraktionierend destilliert. Man erhält 49%iges DMAA aus der azeotropen Destillation.

| **Messzeitpunkt** | **JFZ** | **L∗** | **a** | **b** |
|---|---|---|---|---|
| direkt nach Destillation | 0,0 | 100,1 | -0,3 | 0,9 |
| Dunkellagerung 4 Tage | 0,0 | 100,5 | -0,2 | 0,5 |
| Dunkellagerung 10 Tage | 0,0 | 100,3 | -0,3 | 0,7 |
| Dunkellagerung 30 Tage | 0,3 | 100,1 | -1,0 | 2,4 |

### Beispiel 4:

### Herstellung einer wässrigen DMAA-Lösung aus 2,4-Diamino-6-phenvl-1,3,5-triazin(Benzoguanamin)-stabilisiertem Formaldehyd:

81 g Formalin [37%ige Lösung in Wasser mit 840 ppm 2,4-Diamino-6-phenyl-1,3,5-triazin stabilisiert; 1.00 mol Formaldehyd] werden vorgelegt und 77 g Dimethylaminlösung (60%ige Lösung in Wasser; 1.02 mol Dimethylamin) innerhalb von 20 min zugetropft (T < 35 °C). Die Lösung wird auf ca. 20 °C gekühlt, 27 g (1.00 mol) Blausäure portionsweise zugegeben (T < 35 °C) und 1 h bei ca. 20 °C nachgerührt. Die Produktlösung enthält 45% DMAA in Wasser, die ohne zusätzliche Aufarbeitung als Rohstoff für aufbauende Synthesen weiter verwendet werden kann.

| **Messzeitpunkt** | **JFZ** | **L*** | **a** | **b** |
|---|---|---|---|---|
| direkt nach Synthese | 0,0 | 100,3 | -0,1 | 0,2 |
| Dunkellagerung 4 Tage | 0,0 | 100,4 | -0,2 | 0,5 |
| Dunkellagerung 10 Tage | 0,3 | 99,4 | -0,5 | 1,2 |
| Dunkellagerung 30 Tage | 0,6 | 99,8 | -1,7 | 4,4 |

**Tabelle:**

| Veränderung der Farbe im Zeitverlauf 5 ml einer wässrigen DMAA-Lösung werden in einem verschlossenen Glasgefäß im Dunkeln gelagert. | | | | |
|---|---|---|---|---|
| Lösung aus Beispiel: | JFZ unmittelbar nach Synthese | JFZ nach 4 Tagen | JFZ nach 10 Tagen | JFZ nach 30 Tagen |
| Nr. 1 | 0.2 | 0.3 | 0.5 | 1.4 |
| Nr. 2 | 0.1 | 0.1 | 0.4 | 1.1 |
| Nr. 3 | 0.0 | 0.0 | 0.0 | 0.3 |
| Nr. 4 | 0.0 | 0.0 | 0.3 | 0.6 |

Die Messwerte zeigen deutlich die ausgezeichnete Farbstabilität der erfindungsgemäss herstellten DMAA-Lösung, die fast genauso gut ist wie die einer zusätzlich destillativ gereinigten Lösung.

## Patentansprüche

1. Verfahren zur Herstellung einer farbstabilen, wässrigen Dimethylaminoacetonitril-Lösung ausgehend von einer Formaldehyd-Quelle, Dimethylamin und Blausäure, wobei das Reaktionsgemisch bereits vor der Umsetzung 2,4-Diamino-6-phenyl-1,3,5-triazin (Benzoguanamin) in einer Konzentration von 0.001 bis 5 Gew.-%, bezogen auf Formaldehyd, enthält und die Ausgangssubstanzen stöchiometrisch oder in einem Verhältnis von 0.98 - 1.02 Mol Formaldehyd und 0.95 - 1.10 Mol Dimethylamin je Mol Blausäure eingesetzt werden.

2. Verfahren nach Anspruch 1, wobei die Konzentration an 2,4-Diamino-6-phenyl-1,3,5-triazin zwischen 0.05 und 0.7 Gew.-% liegt.

3. Verfahren nach Anspruch 1 oder 2, wobei die Edukte in einem Zeitraum von 5 min bis 5 h miteinander reagieren.

4. Verfahren nach Anspruch 1 oder 2, wobei die Edukte in einem kontinuierlich betriebenen Herstellprozess zusammengeführt werden.

5. Verfahren nach den Ansprüchen 1 bis 4, wobei die Reaktionstemperatur zwischen 0 und 50 °C liegt.

6. Verfahren nach den Ansprüchen 1 bis 4, wobei die Reaktionstemperatur zwischen 25 und 35 °C liegt.

7. Verfahren nach den Ansprüchen 1 bis 6, wobei es sich bei der Formaldehyd-Quelle um eine wässrige Formaldehyd-Lösung (Formalin) handelt.

8. Verfahren nach den Ansprüchen 1 bis 6, wobei es sich bei der Formaldehyd-Quelle um Paraformaldehyd handelt.

9. Verfahren nach den Ansprüchen 1 bis 8, wobei die Konzentration an Dimethylamin in der wässrigen Dimethylamin-Eduktlösung zwischen 20 und 80 Gew.-% liegt.

10. Verfahren nach den Ansprüchen 1 bis 8, wobei die Konzentration an Dimethylamin in der wässrigen Dimethylamin-Eduktlösung zwischen 35 und 65 Gew.-% liegt.

11. Wässrige Dimethylaminoacetonitril-Lösung, enthaltend 2,4-Diamino-6-phenyl-1,3,5-triazin in einer Konzentration von 0.001 bis 2.0 Gew.-%, bezogen auf das Dimethylaminoacetonitril.

12. Wässrige Dimethylaminoacetonitril-Lösung, enthaltend 2,4-Diamino-6-phenyl-1,3,5-triazin in einer Konzentration von 0.01 bis 0.25 Gew.-%, bezogen auf das Dimethylaminoacetonitril.

13. Verwendung von 2,4-Diamino-6-phenyl-1,3,5-triazin (Benzoguanamin) zur Herstellung farbstabiler wässriger Dimethylaminoacetonitril-Lösungen.

## Claims

1. Process for preparing a colour-stable, aqueous dimethylaminoacetonitrile solution from a formaldehyde source, dimethylamine and hydrocyanic acid, wherein the reaction mixture contains 2,4-diamino-6-phenyl-1,3,5-triazine (benzoguanamine) in a concentration of from 0.001 to 5% by weight, based on formaldehyde, prior to the reaction and the starting substances are used stoichiometrically or in a ratio of 0.98 - 1.02 mol of formaldehyde and 0.95 - 1.10 mol of dimethylamine per mol of hydrocyanic acid.

2. Process according to Claim 1, wherein the concentration of 2,4-diamino-6-phenyl-1,3,5-triazine is from 0.05 to 0.7% by weight.

3. Process according to Claim 1 or 2, wherein the starting materials react with one another over a period of from 5 minutes to 5 hours.

4. Process according to Claim 1 or 2, wherein the starting materials are combined in a continuously operated production process.

5. Process according to any of Claims 1 to 4, wherein the reaction temperature is from 0 to 50°C.

6. Process according to any of Claims 1 to 4, wherein the reaction temperature is from 25 to 35°C.

7. Process according to any of Claims 1 to 6, wherein the formaldehyde source is an aqueous formaldehyde solution (formalin).

8. Process according to any of Claims 1 to 6, wherein the formaldehyde source is paraformaldehyde.

9. Process according to any of Claims 1 to 8, wherein the concentration of dimethylamine in the-aqueous dimethylamine starting solution is from 20 to 80% by weight.

10. Process according to any of Claims 1 to 8, wherein the concentration of dimethylamine in the aqueous dimethylamine starting solution is from 35 to 65% by weight.

11. Aqueous dimethylaminoacetonitrile solution containing 2,4-diamino-6-phenyl-1,3,5-triazine in a concentration of from 0.001 to 2.0% by weight, based on the dimethylaminoacetonitrile.

12. Aqueous dimethylaminoacetonitrile solution containing 2,4-diamino-6-phenyl-1,3,5-triazine in a concentration of from 0.01 to 0.25% by weight, based on the dimethylaminoacetonitrile.

13. Use of 2,4-diamino-6-phenyl-1,3,5-triazine (benzoguanamine) for preparing colour-stable aqueous dimethylaminoacetonitrile solutions.

## Revendications

1. Procédé pour la préparation d'une solution aqueuse, de couleur stable, de diméthylaminoacétonitrile à partir d'une source de formaldéhyde, de diméthylamine et d'acide cyanhydrique, dans lequel le mélange réactionnel contient déjà avant la réaction de la 2,4-diamino-6-phényl-1,3,5-triazine (benzoguanamine) à une concentration de 0,001 à 5% en poids par rapport au formaldéhyde et les substances de départ sont utilisées en quantités stoechiométriques ou dans un rapport de 0,98 - 1,02 mole de formaldéhyde et de 0,95 - 1,10 mole de diméthylamine par mole d'acide cyanhydrique.

2. Procédé selon la revendication 1, dans lequel la concentration en 2,4-diamino-6-phényl-1,3,5-triazine est comprise entre 0,05 et 0,7% en poids.

3. Procédé selon la revendication 1 ou 2, dans lequel les produits de départ sont mis à réagir ensemble pendant une durée de 5 minutes à 5 heures.

4. Procédé selon la revendication 1 ou 2, dans lequel les produits de départ sont combinés dans un procédé de préparation effectué en continu.

5. Procédé selon les revendications 1 à 4, dans lequel la température de réaction est comprise entre 0 et 50°C.

6. Procédé selon les revendications 1 à 4, dans lequel la température de réaction est comprise entre 25 et 35°C.

7. Procédé selon les revendications 1 à 6, dans lequel la source de formaldéhyde est une solution aqueuse de formaldéhyde (formaline).

8. Procédé selon les revendications 1 à 6, dans lequel la source de formaldéhyde est le paraformaldéhyde.

9. Procédé selon les revendications 1 à 8, dans lequel la concentration en diméthylamine dans la solution aqueuse de départ de diméthylamine est comprise entre 20 et 80% en poids.

10. Procédé selon les revendications 1 à 8, dans lequel la concentration en diméthylamine dans la solution aqueuse de départ de diméthylamine est comprise entre 35 et 65% en poids.

11. Solution aqueuse de diméthylaminoacétonitrile, contenant de la 2,4-diamino-6-phényl-1,3,5-triazine à une concentration de 0,001 à 2,0% en poids par rapport au diméthyl-aminoacétonitrile.

12. Solution aqueuse de diméthylaminoacétonitrile, contenant de la 2,4-diamino-6-phényl-1,3,5-triazine à une concentration de 0,01 à 0,25% en poids par rapport au diméthylaminoacétonitrile.

13. Utilisation de la 2,4-diamino-6-phényl-1,3,5-triazine (benzoguanamine) pour la préparation de solutions aqueuses de couleur stable de diméthylaminoacétonitrile.
